# EUROPÄISCHE PATENTSCHRIFT

(11) **EP 0 844 224 B1**
(45) Veröffentlichungstag und Bekanntmachung des Hinweises auf die Patenterteilung: **26.04.2000**
(21) Anmeldenummer: 97113718.7
(22) Anmeldetag: 08.08.1997
(51) Int. Cl.: C07C 4/06, C10G 11/05

(54) **Verfahren zum Erzeugen von C3- und C4- Olefinen aus einem C4- bis C7-Olefine enthaltenden Einsatzgemisch**
Process for the preparation of C3 and C4 olefins from a feedstock comprising C4 to C7 olefins
Procédé pour la préparation d'oléfines en C3 et C4 à partir d'une charge contenant des oléfines en C4 à C7

(30) Priorität: 26.11.1996 DE 19648795
(43) Veröffentlichungstag der Anmeldung: 27.05.1998
(73) Patentinhaber: Metallgesellschaft Aktiengesellschaft, 60325 Frankfurt am Main (DE)
(72) Erfinder: Moeller, Friedrich-Wilhelm, Dr., 61381 Friedrichsdorf (DE); Koenig, Peter, Dr., 60439 Frankfurt am Main (DE); Higman, Christopher, 65824 Schwalbach (DE); Holtmann, Hans-Dieter, Dr., 59199 Boenen (DE)

(56) Entgegenhaltungen:
- EP-A- 0 305 720

## Beschreibung

Die Erfindung betrifft ein Verfahren zum Erzeugen von C₃- und C₄-Olefinen aus einem C₄- bis C₇-Olefine enthaltenden Einsatzgemisch durch Umsetzen des Einsatzgemisches bei Temperaturen von 380 bis 700°C an einem körnigen Zeolith-Katalysator.

Ein solches Verfahren ist aus dem US-Patent 5 059 735 bekannt. Hierbei gibt man dem Einsatzgemisch eine große Menge an Propan zu, bevor man es über den Katalysator leitet, und man erzeugt neben C₂- bis C₄-Olefinen eine erhebliche Menge an C₆₊-Aromaten. Der Erfindung liegt die Aufgabe zugrunde, das bekannte Verfahren so abzuwandeln, daß das Produkt reich an Propylen ist. Gleichzeitig soll das Verfahren möglichst kostengünstig durchgeführt werden. Erfindungsgemäß gelingt dies dadurch, daß man das Einsatzgemisch verdampft und mit Wasserdampf mischt, wobei man ein Gewichtsverhältnis H₂O : Kohlenwasserstoffe im Bereich von 0,5 : 1 bis 3 : 1 einstellt, daß man das wasserdampfhaltige Einsatzgemisch mit einer Eingangstemperatur im Bereich von 380 bis 700°C in einen Reaktor leitet, der eine Schüttung aus körnigem, formselektivem Zeolith-Katalysator enthält, wobei der Zeolith vom Pentasil-Typ ist und ein Atomverhältnis Si : Al im Bereich von 10 : 1 bis 200 : 1 aufweist, und daß man aus der Schüttung und aus dem Reaktor ein Produktgemisch abzieht, dessen Temperatur 20 bis 80°C niedriger als die Eingangstemperatur ist und dessen summierter Gehalt an Propylen und Butenisomeren mindestens 60 Gew.-% und vorzugsweise mindestens 70 Gew.-% der olefinischen Bestandteile des Einsatzgemisches beträgt. Üblicherweise enthält das Produktgemisch auch noch Ethylen, und zwar 5 bis 20 Gew.-%, bezogen auf die olefinischen Bestandteile im Einsatzgemisch. Vorzugsweise liegt die Temperatur des Produktgemisches um 30 bis 50°C niedriger als die Eingangstemperatur. Der Katalysator ist an sich bekannt und z. B. in EP-B-0 369 364 beschrieben.

Es empfiehlt sich, im Reaktor bei relativ niedrigen Drücken im Bereich von 0,2 bis 3 bar zu arbeiten. Üblicherweise liegen die Drücke im Reaktor im Bereich von 0,6 bis 1,5 bar.

Die Zusammensetzung des Einsatzgemisches kann innerhalb weiter Grenzen variieren, dabei empfiehlt es sich, den Aromatengehalt, wasserfrei gerechnet, auf höchstens 5 Gew.-% und vorzugsweise höchstens 2 Gew.-% einzustellen. Dies ist empfehlenswert, weil ein höherer Aromatengehalt durch Kohlenstoffablagerungen zum vorzeitigen Desaktivieren des Katalysators führt. Ferner ist es zweckmäßig, wenn das Einsatzgemisch frei von Komponenten ist, die dreifache C-C-Bindungen oder konjugierte Doppelbindungen haben, da sie ebenfalls den Katalysator desaktivieren. Geeignete Einsatzgemische sind z. B. in der Raffinerie anfallende Gasgemische, die eventuell hydrierend vorbehandelt werden müssen.

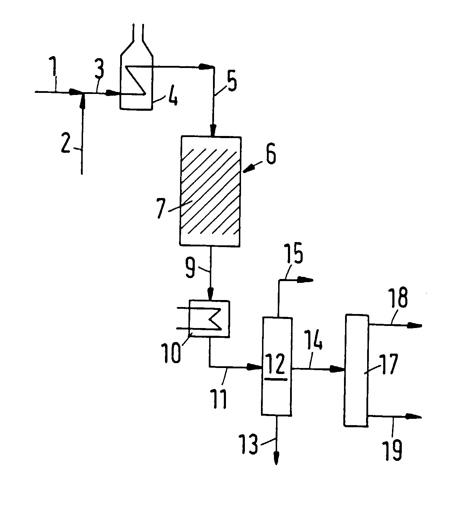

Ausgestaltungsmöglichkeiten des Verfahrens werden mit Hilfe der Zeichnung erläutert. Die Zeichnung zeigt ein Fließschema des Verfahrens.

Verdampftes Einsatzgemisch wird in der Leitung (1) herangeführt und mit Wasserdampf aus der Leitung (2) gemischt. Das Gemisch wird dann in der Leitung (3) durch einen Erhitzer (4) geführt, der vorzugsweise als gefeuerter Erhitzer ausgebildet ist. In der Leitung (5) weist das wasserdampfhaltige Einsatzgemisch eine Temperatur im Bereich von 380 bis 700°C und vorzugsweise 400 bis 600°C auf. Mit dieser Einsatztemperatur wird es in den Reaktor (6) geleitet. Im Gemisch der Leitung (5) beträgt das Gewichtsverhältnis H₂O : Kohlenwasserstoffen 0,5 : 1 bis 3 : 1 und vorzugsweise mindestens 1 : 1.

Im Reaktor (6) ist der körnige Zeolith-Katalysator in Form einer Schüttung (7) angeordnet. Die Korngrößen des Katalysators liegen üblicherweise im Bereich von 1 bis 8 mm. Der Zeolith ist vom Pentasil-Typ, und er hat formselektive Eigenschaften. Wichtig für den Zeolith-Katalysator ist, daß die Primärkristallite des Alumosilikats eine enge Körnungsverteilung mit Durchmessern im Bereich von 0,1 bis 0,9 µm haben. Die BET-Oberfläche liegt üblicherweise im Bereich von 300 bis 600 m²/g und das Porenvolumen (nach der Quecksilberporosimetrie) beträgt etwa 0,3 bis 0,8 cm³/g. Als Bindemittel zum Zusammenhalten der Primärkristallite wird vorzugsweise Aluminiumoxidhydrat verwendet.

Die Umsetzung im Reaktor (6) erfolgt vorzugsweise adiabatisch, so daß die Temperatur des umzusetzenden Gemisches beim Durchströmen der Schüttung (7) sinkt. Dadurch weist das Produktgemisch, das man in der Leitung (9) abzieht, eine Temperatur auf, die 20 bis 80°C und zumeist 30 bis 50°C niedriger ist als die Eingangstemperatur in der Leitung (5). Üblicherweise liegt die Ausgangstemperatur in der Leitung (9) bei mindestens 350°C. Das Hauptprodukt des Produktgemisches der Leitung (9) ist Propylen. 80 bis 100 Gew.-% der Olefine im Einsatzgemisch der Leitung (5) werden im Reaktor (6) umgesetzt.

Im Kühler (10) wird das Produktgemisch der Leitung (9) auf Temperaturen von etwa 30 bis 60°C gekühlt, so daß Wasser und Benzin auskondensieren. Das kondensathaltige Gemisch wird in der Leitung (11) zu einem Abscheider (12) geführt. Aus dem Abscheider (12) zieht man durch die Leitung (13) Wasser ab, in der Leitung (14) erhält man eine organische Flüssigphase, und durch die Leitung (15) wird ein Produktgas abgezogen. Das Produktgas enthält C₂- bis C₄-Olefine und daneben noch einen geringen Anteil an Paraffinen. Um die Wertstoffe abzutrennen, insbesondere Ethylen und Propylen, gibt man das Gas der Leitung (15) einer nicht dargestellten Trenneinrichtung auf, die an sich bekannt ist.

Die organische Flüssigphase der Leitung (14) wird in der Destillationskolonne (17) in C₃- und C₄-Olefinfraktion, abgezogen durch die Leitung (18), und in Benzin, abgezogen durch die Leitung (19), aufgetrennt. Das Kopfprodukt, das in der Leitung (18) abgezogen wird, enthält üblicherweise noch kleine Mengen an gesättigten Kohlenwasserstoffen. Im Benzin der Leitung (19) sind nur geringe Mengen (höchstens 5 Gew.-%) Aromaten enthalten. Das propylenhaltige Produkt der Leitung (18) wird üblicherweise ebenfalls einer nicht dargestellten Trenneinrichtung zugeführt, um z. B. destillativ oder adsorptiv gewünschte Wertstoffe zu gewinnen. Bei diesen Wertstoffen handelt es sich neben Propylen vor allem um Ethylen, n-Buten-1 und Isobutylen. Anfallendes n-Buten-2, für das keine Verwendung besteht, kann in den Reaktor (6) zurückgeführt werden.

### Beispiele:

Im Labor wird mit einer der Zeichnung entsprechenden Anlage gearbeitet. Der Katalysator ist in allen Fällen der gleiche, er wird in Form von Extrudaten der Abmessungen 1,5 x 3 mm angewandt. Der Katalysator ist im einzelnen im Patent von Südchemie EP-B-0 369 364 in den Beispielen 1 bis 5 beschrieben.

### Beispiel 1:

Es wird folgendes Einsatzgemisch hergestellt:

| | |
|---|---|
| n-Penten-1 (C₅H₁₀) | 40 Gew.-% |
| n-Hexen-1 (C₆H₁₂) | 20 Gew.-% |
| n-Pentan (C₅H₁₂) | 20 Gew.-% |
| n-Hexan (C₆H₁₄) | 20 Gew.-% |

Pro kg der Olefine gibt man dem Einsatzgemisch 1,5 kg Wasser zu, wärmt das Gemisch auf 460°C an und leitet es bei einem Druck von 0,5 bar durch den Reaktor (6), der die Katalysatorschüttung (7) enthält. Die Belastung pro Stunde beträgt 1 kg Kohlenwasserstoffe pro kg Katalysator. Das Produktgemisch, welches man mit einer Temperatur von 435°C aus dem Reaktor abzieht, enthält

| | | |
|---|---|---|
| | Ethylen | 4,8 Gew.-% |
| | Propylen | 23,5 Gew.-% |
| | Isobutylen | 7,7 Gew.-% |
| | n-Buten-1 | 2,0 Gew.-% |
| | c-Buten-2 | 2,9 Gew.-% |
| | t-Buten-2 | 3,6 Gew.-% |
| Insgesamt | C₂ - C₄-Olefine | 44,5 Gew.-% |

### Beispiel 2:

Das Einsatzgemisch des Beispiels 1 wird zusammen mit 1,5 kg Wasser pro kg Kohlenwasserstoffe auf 500°C vorgewärmt und bei einem Druck von 2 bar und der Belastung von 3 kg/kg/h (wasserfrei gerechnet) durch die Katalysatorschüttung geleitet. Das Produktgemisch in der Leitung (9) ist auf 470°C abgekühlt und enthält 41,6 Gew.-% Olefine der Zusammensetzung:

| | |
|---|---|
| Ethylen | 9,9 Gew.-% |
| Propylen | 48,6 Gew.-% |
| Butene | 41,5 Gew.-%. |

### Beispiel 3:

Das Beispiel 2 wird so abgewandelt, daß nunmehr bei einem Druck im Reaktor (6) von 1,3 bar und einer Belastung des Katalysators von 2 kg/kg/h (wasserfrei gerrechnet) gearbeitet wird. Die Temperatur in der Leitung (9) beträgt 462°C und nach dem Kühler (10) ist die Temperatur in der Leitung (11) auf 35°C gesunken. Nach der Abtrennung von Wasser, das eine kleine Menge organischer Verbindungen enthält, verbleiben als flüssiges und gasförmiges Produkt

| | | Olefine | Paraffine | Naphtene | Aromaten | Summe |
|---|---|---|---|---|---|---|
| **1. C2-C4-Olefine:** | | | | | | |
| C2 | (Gew.-%) | 7,22 | | | | |
| C3 | (Gew.-%) | 26,07 | | | | |
| C4 | (Gew.-%) | 18,33 | | | | |
| Summe | (Gew.-%) | 51,62 | | | | 51,62 |

| **2. Brenngas:** | | | | | | |
|---|---|---|---|---|---|---|
| C1 | (Gew.-%) | | 0,13 | | | |
| C2 | (Gew.-%) | | 0,31 | | | |
| C3 | (Gew.-%) | | 2,10 | | | |
| C4 | (Gew.-%) | | 2,05 | | | |
| Summe | (Gew.-%) | | 4,59 | | | 4,59 |

| **3. Benzin:** | | | | | | |
|---|---|---|---|---|---|---|
| C5 | (Gew.-%) | 5,17 | 16,19 | 0,51 | | |
| C6 | (Gew.-%) | 1,13 | 15,94 | 0,80 | 0,40 | |
| C7+ | (Gew.-%) | 0,62 | 0,85 | 0,45 | 1,73 | |
| Summe | (Gew.-%) | 6,92 | 32,98 | 1,76 | 2,13 | 43,79 |
| **Gesamt** | (Gew.-%) | | | | | 100,00 |

### Beispiel 4:

Es wird folgendes Einsatzgemisch vorbereitet und im übrigen wie im Beispiel 3 gearbeitet:

| | |
|---|---|
| t-Buten-2 | 9,2 Gew.-% |
| n-Penten-1 | 35,9 Gew.-% |
| n-Hexen-1 | 18,3 Gew.-% |
| n-Pentan | 18,3 Gew.-% |
| n-Hexan | 18,3 Gew.-% |

Nach Entfernen des Wassers durch die Leitung (13) erhält man in den zusammengefaßten Leitungen (14) und (15) eine C₂- bis C₄-Olefinfraktion folgender Zusammensetzung:

| | |
|---|---|
| Ethylen | 12,1 Gew.-% |
| Propylen | 49,7 Gew.-% |
| Isobutylen | 17,5 Gew.-% |
| n-Buten-1 | 5,3 Gew.-% |
| c-Buten-2 | 7,1 Gew.-% |
| t-Buten-2 | 8,3 Gew.-% |

Diese Olefinfraktion macht 57,2 Gew.-% der organischen Substanzen der zusammengefaßten Leitungen (14) und (15) aus.

## Patentansprüche

1. Verfahren zum Erzeugen von C₃- und C₄-Olefinen aus einem C₄- bis C₇-Olefine enthaltenden Einsatzgemisch durch Umsetzen des Einsatzgemisches bei Temperaturen von 380 bis 700°C an einem körnigen Zeolith-Katalysator, dadurch gekennzeichnet, daß man das Einsatzgemisch verdampft und mit Wasserdampf mischt, wobei man ein Gewichtsverhältnis H₂O : Kohlenwasserstoffe im Bereich von 0,5 : 1 bis 3 : 1 einstellt, daß man das wasserdampfhaltige Einsatzgemisch mit einer Eingangstemperatur im Bereich von 380 bis 700°C in einen Reaktor leitet, der eine Schüttung aus körnigem, formselektivem Zeolith-Katalysator enthält, wobei der Zeolith vom Pentasil-Typ ist und ein Atomverhältnis Si : Al im Bereich von 10 : 1 bis 200 : 1 aufweist, und daß man aus der Schüttung und aus dem Reaktor ein Produktgemisch abzieht, dessen Temperatur 20 bis 80°C niedriger als die Eingangstemperatur ist und dessen summierter Gehalt an Propylen und Butenisomeren mindestens 60 Gew.-% der olefinischen Bestandteile des Einsatzgemisches beträgt.

2. Verfahren nach Anspruch 1, dadurch gekennzeichnet, daß man im Reaktor bei einem Druck im Bereich von 0,2 bis 3 bar arbeitet.

3. Verfahren nach Anspruch 1 oder 2, dadurch gekennzeichnet, daß man den Aromatengehalt im Einsatzgemisch, wasserfrei gerechnet, auf höchstens 5 Gew.-% einstellt.

4. Verfahren nach Anspruch 1 oder einem der folgenden, dadurch gekennzeichnet, daß man das aus dem Reaktor abgezogene Produktgemisch auf Temperaturen von etwa 30 bis 60°C kühlt, Wasser auskondensiert und abtrennt, ein Produktgas von einer organischen Flüssigphase trennt und aus der organischen Flüssigphase eine Kohlenwasserstofffraktion abtrennt, die reich an Propylen, Isobutylen und n-Butenen ist.

## Claims

1. A process for producing C₃- and C₄-olefins from a feed mixture containing C₄- to C₇-olefins by reacting the feed mixture at temperatures of 380 to 700°C on a granular zeolite catalyst, characterised in that the feed mixture is evaporated and is mixed with steam, with a weight ratio of H₂O : hydrocarbons in the range of 0.5 : 1 to 3 : 1 being set, that the steam-containing feed mixture is passed at an entry temperature in the range from 380 to 700°C into a reactor which contains a bed of granular, form-selective zeolite catalyst, the zeolite being of the pentasil type and having an atomic ratio of Si : Al in the range from 10 : 1 to 200 : 1, and that a product mixture is withdrawn from the bed and from the reactor, the temperature of which mixture is 20 to 80°C lower than the entry temperature and the totalled content of propylene and butene isomers of which is at least 60% by weight of the olefinic constituents of the feed mixture.

2. A process according to Claim 1, characterised in that the reactor is operated at a pressure in the range from 0.2 to 3 bar.

3. A process according to Claim 1 or 2, characterised in that the aromatics content in the feed mixture, calculated in anhydrous state, is set to at most 5% by weight.

4. A process according to Claim 1 or one of the following claims, characterised in that the product mixture withdrawn from the reactor is cooled to temperatures of about 30 to 60°C, water is condensed out and separated off, a product gas is separated from an organic liquid phase and a hydrocarbon fraction which is rich in propylene, isobutylene and n-butenes is separated off from the organic liquid phase.

## Revendications

1. Procédé pour la préparation d'oléfines en C₃ et en C₄ à partir d'un mélange de mise en oeuvre contenant des oléfines en C₄-C₇, par mise en réaction du mélange de mise en oeuvre à des températures de 380 à 700°C sur un catalyseur de zéolithe granuleux, caractérisé en ce qu'on évapore le mélange de mise en oeuvre et on le mélange avec de la vapeur d'eau, dans lequel on règle un rapport pondéral H₂O : hydrocarbures dans le domaine de 0,5 : 1 à 3 : 1, en ce qu'on guide le mélange de mise en oeuvre contenant de la vapeur d'eau avec une température d'entrée dans le domaine de 380 à 700°C dans un réacteur qui contient un catalyseur de zéolithe granuleux en vrac de forme sélective, la zéolithe étant de type Pentasil et présentant un rapport atomique Si : Al dans le domaine de 10 : 1 à 200 : 1, et en ce qu'on extrait de la masse en vrac et du réacteur, un mélange de produit dont la température est inférieure de 20 à 80°C à la température d'entrée et dont la teneur additionnée en isomères du propylène et du butène représente au moins 60% en poids des constituants oléfiniques du mélange de mise en oeuvre.

2. Procédé selon la revendication 1, caractérisé en ce qu'on travaille dans le réacteur sous une pression dans le domaine de 0,2 à 3 bar.

3. Procédé selon la revendication 1 ou 2, caractérisé en ce qu'on règle la teneur du mélange de mise en oeuvre en composés aromatiques, calculée à l'état anhydre, à une valeur maximale de 5% en poids.

4. Procédé selon la revendication 1 ou selon une des revendications suivantes, caractérisé en ce qu'on refroidit le mélange de produit extrait du réacteur à des températures d'environ 30 à 60°C, on condense et on sépare l'eau, on sépare un produit gazeux d'une phase liquide organique et on sépare de la phase liquide organique une fraction d'hydrocarbure qui est riche en propylène, en isobutylène et en n-butènes.
